# EUROPÄISCHE PATENTSCHRIFT

(11) **EP 0 627 208 B1**
(45) Veröffentlichungstag und Bekanntmachung des Hinweises auf die Patenterteilung: **25.09.1996**
(21) Anmeldenummer: 94108262.0
(22) Anmeldetag: 28.05.1994
(51) Int. Cl.: A61F 13/10

(54) **Epicondylitis-Bandage**
Epicondylitis bandage
Bandage pour l'épicondylite

(30) Priorität: 04.06.1993 DE 4318575
(43) Veröffentlichungstag der Anmeldung: 07.12.1994
(73) Patentinhaber: SCHÜTT & GRUNDEI ORTHOPÄDIETECHNIK GmbH, D-23556 Lübeck (DE)
(72) Erfinder: Grundei, Hans, Dr., D-23558 Lübeck (DE); Etspüler, Rolf, Dr., D-23562 Lübeck (DE); Schmelzer, Ulrich, Dr., D-23568 Lübeck (DE)
(74) Vertreter: Fuchs, Luderschmidt & Partner Patentanwälte

(56) Entgegenhaltungen:
- EP-A- 0 375 809
- DE-C- 4 129 675
- US-A- 1 374 669

## Beschreibung

Die Erfindung betrifft eine Epicondylitis-Bandage, die beispielsweise Anwendung in der Therapie von Entzündungen der Sehnenansätze im Ellbogenbereich findet.

Ziel einer Therapie ist eine Reduzierung der muskulösen Zugkräfte auf die Sehnenansätze am Ellbogenknochen und eine Stimulierung des Heilungsprozesses durch kontinuierliche Druckmassage der Sehnenansätze.

Eine röhrenförmige Epicondylitis-Bandage ist beispielsweise bekannt aus der DE 35 05 369 A1 und aus der jüngst veröffentlichten DE 41 29 675 C1 der Anmelderin. In der letztgenannten Druckschrift ist zwischen dem Unterarmabschnitt und dem Oberarmabschnitt ein faltenbalgförmig ausgebildetes Übergangsstück im Bereich der Ellenbogenbeuge vorgesehen. Bei der Epicondylitis-Bandage gemäß der DE 35 05 369 A1 findet sich ein solches faltenbalgförmiges Element auf seiten des Ellenbogenhakens.

Bei diesen mehr oder weniger geschlossenen Formen von Epicondylitis-Bandagen können sich Probleme hinsichtlich des Tragekomforts einstellen, selbst wenn das elastische Material, aus dem diese Bandagen bestehen können, atmungsaktiv ist.

Vor diesem Hintergrund ist es daher die Aufgabe der vorliegenden Erfindung, eine Epicondylitis-Bandage anzugeben, die bei erhöhtem Tragekomfort einen hinreichend sicheren Sitz gewährleistet.

Gelöst wird diese Aufgabe durch eine Epicondylitis-Bandage gemäß dem Anspruch 1. Weitere vorteilhafte Ausgestaltungen ergeben sich aus den Unteransprüchen.

Die erfindungsgemäße Epicondylitis-Bandage besteht also aus einem elastischen Material in im wesentlichen röhrenförmiger Ausbildung. Sie kann also in einfacher Weise über die Hand und den Unterarm bis zum Ellenbogen übergestreift werden. Sie weist einen Unterarmabschnitt und einen Oberarmabschnitt auf. Im Bereich der Ellenbogenbeuge ist ein Ausschnitt im elastischen Material vorgesehen, ebenso wie gegenüberliegend im Bereich des Ellenbogenhakens. Eine derart ausgebildete Bandage hätte zwar einen erhöhten Tragekomfort, böte aber noch keinen hinreichend sicheren Sitz am Ellenbogen. Erfindungsgemäß ist daher ein weiterer Ausschnitt im Oberarmabschnitt vorgesehen, derart, daß zwischen dem Ausschnitt für den Ellenbogenhaken und dem weiteren Ausschnitt ein Spannband aus dem elastischen Material der Bandage mit einer Breite zwischen 10 und 20 mm gebildet ist.

Die erfindungsgemäße Epicondylitis-Bandage sitzt faltenfrei und verrutscht nicht, da das besagte Spannband sich hinter dem Ellenbogenhaken dort anlegt. Der weitere Ausschnitt sowie die beiden größeren Ausschnitte für die Ellenbogenbeuge und den Ellenbogenhaken erlauben eine weitgehend ungestörte Armbeugung.

Gemäß einer vorteilhaften Weiterbildung ist zumindest lateral, vorzugsweise lateral und medial, längs des Oberarmabschnittes und des Bereiches der Ausschnitte für das Ellenbogengelenk jeweils ein Versteifungselement in einer Textiltasche vorgesehen. Das Versteifungselement kann beispielsweise die Form einer Spirale haben.

Die Heilwirkung der erfindungsgemäßen Epicondylitis-Bandage kann erhöht werden dadurch, daß gemäß einer vorteilhaften Weiterbildung im lateralen und/oder medialen Unterarmabschnitt in einer Textiltasche eine Kugel enthalten ist, welche für die Massage des Sehnenspiegels sorgt. Diese Anordnung ist als solche bekannt aus der DE 41 29 675 C1.

Schließlich kann ein Spanngurt vorgesehen sein, der mittels eines Schnellverschlusses um den Unterarmabschnitt spannbar ist.

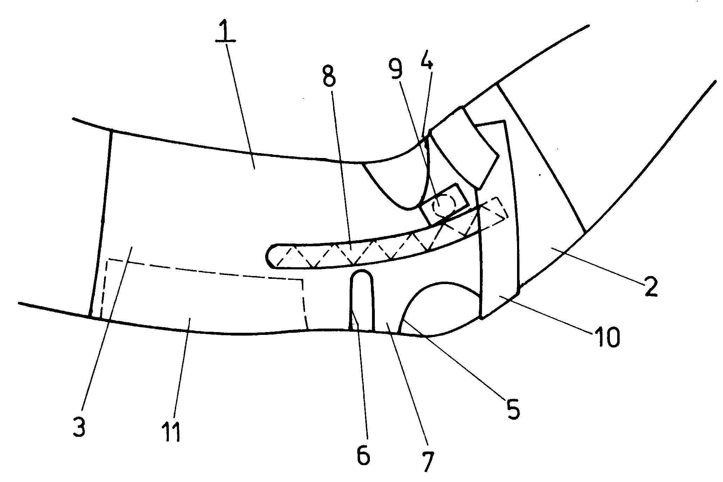

Die Erfindung wird anhand der einzigen Zeichnungsfigur näher erläutert, eine Ausführungsform der Bandage im angelegten Zustand von lateral her gesehen.

Die Bandage 1 weist einen Unterarmabschnitt 2 sowie einen Oberarmabschnitt 3 auf. Im Übergangsbereich dieser beiden Abschnitte ist außen ein Ausschnitt 5 und innen ein Ausschnitt 4 in dem elastischen Material der Bandage 1 vorgesehen. Der Ausschnitt 4 liegt an der Ellenbogenbeuge, der Ausschnitt 5 ist im Bereich des Ellenbogenhakens vorgesehen. Dieser tritt durch den Ausschnitt 5 aus.

Es ist im Oberarmabschnitt 3 ein weiterer Ausschnitt 6 vorgesehen, der so angebracht ist, daß zwischen dem Ausschnitt 6 und dem Ausschnitt 5 für den Ellenbogenhaken ein Spannband 7 entsteht. Dieses ist zwischen 10 und 20 mm breit und legt sich hinter den Ellenbogenhaken an den Oberarm an.

Zur Versteifung ist ein Versteifungselement 8, beispielsweise in Form einer Spirale, in einer Textiltasche untergebracht und reicht vom unteren Ende des Oberarmabschnittes 3 bis über die Ausschnitte 4 und 5 bis ansatzweise in den Unterarmabschnitt 2. Es ist zu erkennen, daß im lateralen Unterarmabschnitt 2 eine Textiltasche angenäht ist, in der eine Kugel 9 gehalten wird. Diese dient zur Massage des Sehnenspiegels.

Schließlich ist um den Unterarmabschnitt 2 noch ein Spanngurt 10 gespannt, dessen Enden mittels eines Schnellverschlusses verschließbar sind.

Zum Anlegen wird die Epicondylitis-Bandage über den Unter- und den Oberarm gestreift, bis die Ausschnitte 4 und 5 am Ellenbogengelenk sitzen. Sodann wird der Oberarmabschnitt 3 mit einem Schnellverschluß 11 verschlossen.

## Patentansprüche

1. Epicondylitis-Bandage (1) aus elastischem Material in im wesentlichen röhrenförmiger Ausbildung mit einem Unterarmabschnitt (2) und einem Oberarmabschnitt (3), gekennzeichnet durch je einem Ausschnitt (4, 5) im Bereich der Ellenbogenbeuge und im Bereich des Ellenbogenhakens sowie einem weiteren Ausschnitt (6) im Oberarmabschnitt (3), derart, daß sich zwischen dem Ausschnitt (5) für den Ellenbogenhaken und dem weiteren Ausschnitt (6) ein Spannband (7) mit einer Breite zwischen 10 und 20 mm aus dem elastischen Material der Bandage bildet.

2. Epicondylitis-Bandage nach Anspruch 1, bei der zumindest lateral längs des unteren Oberarmabschnittes (3) und des Bereiches der Ausschnitte (4, 5) in einer Textiltasche ein Versteifungselement (8) vorgesehen ist.

3. Epicondylitis-Bandage nach Anspruch 2, bei der auch medial ein solches Versteifungselement (8) vorgesehen ist.

4. Epicondylitis-Bandage nach einem der Ansprüche 1 bis 3, bei der im lateralen und/oder medialen Unterarmabschnitt (2) in jeweils einer Textiltasche eine Kugel (9) gehalten ist.

5. Epicondylitis-Bandage nach einem der Ansprüche 1 bis 4, bei der ein Spanngurt (10) vorgesehen ist, der mittels eines Schnellverschlusses um den Unterarmabschnitt (2) gehalten ist.

## Claims

1. Epicondylitis bandage (1) made from resilient material of essentially tubular design having a lower arm section (2) and an upper arm section (3), characterised in each case by a cutout (4, 5) in the region of the elbow bend and in the region of the elbow hook as well as a further cutout (6) in the upper arm section (3), such that a tension band (7) made from the resilient material of the bandage having a width between 10 and 20 mm is formed between the cutout (5) for the elbow hook and the further cutout (6).

2. Epicondylitis bandage according to claim 1, in which a stiffening element (8) in a textile pocket is provided at least laterally along the lower upper arm section (3) and the region of the cutouts (4, 5).

3. Epicondylitis bandage according to claim 2, in which a stiffening element (8) of this type is also provided medially.

4. Epicondylitis bandage according to one of claims 1 to 3, in which a ball (9) in each case in a textile pocket is held in the lateral and/or medial lower arm section (2).

5. Epicondylitis bandage according to one of claims 1 to 4, in which a tension strap (10), which is held around the lower arm section (2) by means of a rapid closure, is provided.

## Revendications

1. Bandage d'épicondyle (1) on matériau élastique dans une réalisation sensiblement tubulaire avec uni portion d'avant-bras (2) et une portion de bras (3), caractérisé par une découpe (4, 5) dans la région du pli du coude et dans la région du crochet du coude ainsi qu'une autre découpe (6) dans la portion de bras (3), de manière qu'entre la découpe (5) pour le crochet du coude et l'autre découpe (6) il se forme une bande de serrage (7), d'une largeur comprise entre 10 et 20 mm, dans le matériau élastique du bandage.

2. Bandage d'épicondyle selon la revendication 1, dans lequel un élément de renfort (8) est prévu au moins latéralement, le long de la portion de bras (3) inférieure et de la zone des découpes (4, 5), dans une poche textile.

3. Bandage d'épicondyle selon la revendication 2, dans lequel il est prévu un tel élément de renfort (8) également en position médiane.

4. Bandage d'épicondyle selon l'une des revendications 1 à 3, dans lequel dans la portion d'avant-bras (2) latérale et/ou médiane une bille (9) est maintenue dans une poche textile.

5. Bandage d'épicondyle selon l'une des revendications 1 à 4, dans lequel est prévue une sangle de serrage (10), qui est maintenue au moyen d' une fermeture rapide autour de la portion d'avant-bras (2).
